# EUROPÄISCHE PATENTANMELDUNG

(11) **EP 1 247 794 A2**
(43) Veröffentlichungstag der Anmeldung: **09.10.2002**
(21) Anmeldenummer: 02007307.8
(22) Anmeldetag: 03.04.2002
(51) Int. Cl.: C07C 67/297, C07C 69/007, C07C 67/29

(54) **Verfahren zur Herstellung von C5-Acetat für die Vitamin A-Synthese**

(30) Priorität: 05.04.2001 DE 10117065
(71) Anmelder: BASF AKTIENGESELLSCHAFT, 67056 Ludwigshafen (DE)
(72) Erfinder: Rheude, Udo, Dr., 67166 Otterstadt (DE); Vicari, Maximilian, Dr., 67117 Limburgerhof (DE); Aquila, Werner, Dr., 68309 Mannheim (DE); Wegner, Günter, Dr., 67354 Römerberg (DE); Niekerken, Jörg, 37603 Holzminden (DE)
(74) Vertreter: Isenbruck, Günter, Dr.

(57) **Zusammenfassung**

Es wird ein Verfahren zur Herstellung von C₅-Acetat für die Vitamin A-Synthese durch Hydroformylierung von 3,4-Diacetoxy-Buten(1), (3,4-DABE) vorgeschlagen, wonach man ein 3,4-DABE einsetzt, das man durch Acetoxylierung von 1,3-Butadien mit Essigsäure und Luft in Gegenwart eines Katalysators und destillative Abtrennung des hierbei als Nebenprodukt gebildeten 3,4-DABE erhalten hat.

## Beschreibung

Die Erfindung betrifft ein Verfahren zur Herstellung des C₅-Acetats für die Vitamin A-Synthese.

Zur Vitamin A-Synthese nach dem Verfahren der BASF werden aus petrochemischen Grundstoffen zunächst ein C₅- und ein C₁₅-Teilstück aufgebaut, die anschließend mit Hilfe der Wittig-Reaktion zu Vitamin A vereinigt werden (vgl. Chem.-Ing.-Techn. 45. Jahrg. 1973, Nr. 10a S. 646-652). Das C₅-Teilstück, für das im folgenden die Bezeichnung C₅-Acetat verwendet wird, ist β-Formylcrotylacetat mit der nachfolgenden Strukturformel

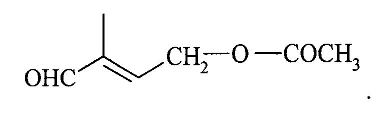

C₅-Acetat wurde bislang ausgehend von Buten(2)-diol-(1,4) durch Veresterung mit Essigsäure und Acetanhydrid und Isomerisierung des hierbei gebildeten Buten(2)-1,4-Diacetats zum 3,4-Diacetoxybuten(1), im folgenden abgekürzt als 3,4-DABE bezeichnet, Hydroformylierung des 3,4-DABE und anschließende Essigsäureabspaltung erhalten.

Die Isomerisierung von Buten(2)-diol-(1,4)-Diacetat zum 3,4-DABE ist beispielsweise in DE-B 24 06 058 beschrieben. Nach dem dort beschriebenen Verfahren wird die Isomerisierung in Gegenwart von metallischem Kupfer und/oder Kupfer(I)- und/oder Kupfer(II)-Verbindungen als Katalysatoren sowie in Gegenwart von Carbonsäuren durchgeführt. Als Carbonsäure wird hierbei zweckmäßig dieselbe Säure eingesetzt, die auch im Diester enthalten ist, der den Ausgangsstoff darstellt, im Falle von Buten(2)-diol-(1,4)-diacetat somit Essigsäure.

Die Hydroformylierung von 3,4-DABE zur Herstellung von C₅-Acetat kann in vorteilhafter Weise in Gegenwart von Rhodiumkatalysatoren erfolgen. Ein derartiges Verfahren ist beispielsweise in DE-A 19 45 479 beschrieben. Danach wird 3,4-DABE mit Kohlenmonoxid und Wasserstoff bevorzugt in stöchiometrischen Mengen bei Temperaturen von 60 bis 120°C, bevorzugt von 80 bis 105°C und bei Drücken von 300 bis 1200 Atmosphären, bevorzugt von 500 bis 700 Atmosphären in Gegenwart von Rhodiumcarbonylkomplexen umgesetzt. Die Rhodiumcarbonylkomplexe können vor der Hydroformylierungsaktion gesondert hergestellt werden oder es können der Hydroformylierung die Ausgangsstoffe hierfür zugeführt werden, wobei sich die Rhodiumcarbonylkomplexe in situ bilden. Die Katalysatorkonzentration beträgt vorzugsweise zwischen 0,00005 bis 0,05 Gew.-%, besonders bevorzugt 0,0001 bis 0,01 % an Rhodium, berechnet als Metall, bezogen auf die Menge des eingesetzten Edukts 3,4-DABE. Die Umsetzung kann ohne Mitverwendung inerter Lösungsmittel wie auch in Gegenwart von inerten Lösungsmitteln wie Kohlenwasserstoffen, beispielsweise Benzol, Cyclohexan, Xylol, Hexan von Ethern wie Diethylether, Tetrahydrofuran oder Dioxan oder von Estern wie Ethylacetat, durchgeführt werden.

Demgegenüber war es Aufgabe der Erfindung, die Raum-Zeit-Ausbeute der Hydroformylierungsreaktion von 3,4-DABE zum C₅-Acetat und somit die Wirtschaftlichkeit des Verfahrens zu verbessern.

Die Lösung geht aus von einem Verfahren zur Herstellung von C₅-Acetat für die Vitamin A-Synthese durch Hydroformylierung von 3,4-Diacetoxy-Buten(1) (3,4-DABE).

Die Erfindung ist dadurch gekennzeichnet, daß man einen 3,4-DABE enthaltenden Strom einsetzt, den man durch Acetoxylierung von 1,3-Butadien mit Essigsäure und Luft in Gegenwart eines Katalysators und destillative Abtrennung des hierbei als Nebenprodukt gebildeten 3,4-DABE erhalten hat.

Es wurde überraschend gefunden, daß 3,4-DABE, das als Nebenprodukt der Acetoxylierung von Butadien anfällt, nicht nur als Wertprodukt für die Weiterverarbeitung zum C₅-Acetat verwendet werden kann, sondern darüber hinaus gegenüber einem ausgehend von Buten(2)-1,4-Diol hergestelltem 3,4-DABE im Verfahren zur Herstellung des C₅-Acetats zu einem wesentlich besseren Ergebnis, insbesondere einer verbesserten Raum-Zeit-Ausbeute, führt.

Ausgehend von 1,3-Butadien wird in großtechnischem Mengen 1,4-Butandiol hergestellt, das ein Grundprodukt der chemischen Industrie ist. Hierzu wird 1,3-Butadien in einem ersten Verfahrensschritt mit Essigsäure und Luft acetoxyliert. Bei dieser Reaktion entsteht neben dem für die Weiterverarbeitung zum 1,4-Butandiol erwünschten 1,4-Diacetoxybuten auch das isomere 3,4-DABE in einem erheblichen Anteil, häufig von 15 oder mehr Gew.-%.
Ein Verfahren zur Acetoxylierung von 1,3-Butadien und zur destillativen Abtrennung des dabei als Nebenprodukt gebildeten 3,4-DABE ist beispielsweise in DE-A 33 09 168 beschrieben. Hiernach wird die Acetoxylierung in Gegenwart von Katalysatoren und bei erhöhten Temperaturen und Drücken durchgeführt. Als Katalysatoren werden vorzugsweise Palladium und/oder Platin enthaltende Katalysatoren aufgeführt, die insbesondere geträgert sind. Die aktive Katalysatormasse kann neben Palladium oder Platin auch weitere Metalle, wie Te, Cu, Sb, Se oder Bi enthalten. Bevorzugt ist ein Temperaturbereich von 80 bis 120°C und ein Druckbereich von 1 bis 100 bar. Aus dem Reaktionsgemisch der Acetoxylierung werden zunächst Leichtsieder, insbesondere nicht umgesetztes 1,3-Butadien, abgetrennt und anschließend die schwersiedende Fraktion, die überwiegend 1,4-DABE und 3,4-DABE enthält, fraktioniert destilliert, vorzugsweise bei Sumpftemperaturen zwischen 150 und 180°C, entsprechend Kolonnendrücken von 60 bis 80 mbar. Dabei wird ein überwiegend 3,4-DABE enthaltender Strom abgetrennt, der nach den Angaben der DE-A 33 09 168 ganz oder teilweise in den Acetoxylierungsreaktor zurückgeführt wird.

Nach dem Verfahren der vorliegenden Erfindung wird der durch destillative Abtrennung aus dem Acetoxylierungsprodukt von 1,3-Butadien erhaltene, 3,4-DABE enthaltende Strom für die Weiterverarbeitung zum C₅-Acetat eingesetzt.

Hierbei gibt es keine Einschränkungen bezüglich der konkreten Verfahrensbedingungen der Acetoxylierungsreaktion. Diese kann besonders bevorzugt, wie vorstehend dargelegt, nach dem Verfahren der DE-A 33 09 168 durchgeführt werden.

Auch bezüglich der destillativen Abtrennung eines 3,4-DABE enthaltenden Stroms aus dem Reaktionsgemisch der Acetoxylierung gibt es grundsätzlich keine Einschränkungen.

Vorteilhaft kann das Reaktionsgemisch der Acetoxylierung eine Destillationskolonne in deren unteren Bereich zugeführt und der 3,4-DABE enthaltende Strom der Destillationskolonne als flüssiger Seitenstrom abgezogen werden.

Die Destillationskolonne wird bevorzugt bei vermindertem Druck betrieben, um Zerfallsoder Umwandlungsreaktionen zu vermeiden. Bevorzugt ist eine Sumpftemperatur im Bereich von 110 bis 190°C, insbesondere von 120 bis 150°C, besonders bevorzugt von 130°C und ein Druck im Bereich von 5 bis 200 mbar, insbesondere von 20 bis 50 mbar, besonders bevorzugt von 30 mbar.

Alternativ zur Abtrennung des 3,4-DABE enthaltenden Stroms als flüssiger Seitenabzug einer Destillationskolonne ist es möglich, zwei hintereinander geschaltete Destillationskolonnen einzusetzen, wobei der Kopfstrom aus der ersten Destillationskolonne der zweiten Destillationskolonne zugeführt wird und aus dem Sumpf der zweiten Destillationskolonne eine 3,4-DABE enthaltender Strom abgezogen wird.

Bezüglich der trennwirksamen Einbauten für die Destillationskolonne(n) gibt es grundsätzlich keine Einschränkungen, besonders bevorzugt sind jedoch strukturierte geordnete Packungen aus metallischen oder keramischen Werkstoffen.

Der destillativ abgetrennte, 3,4-DABE enthaltende Strom weist häufig einen Gewichtsanteil im Bereich von 75 bis 90 Gew.-%, bevorzugt von ca. 85 %, bezogen auf das Gesamtgewicht, an 3,4-DABE auf. Daneben sind häufig in höherem Gewichtsanteil die entsprechenden Monoacetate, d.h. das 3-Hydroxy-4-acetoxy-buten(1) und das 3-Acetoxy-4-hydroxy-buten(1) enthalten. Die beiden vorstehend genannten Komponenten bzw. Gemische hiervon werden nachfolgend mit dem gemeinsamen Begriff 3,4-HABE bezeichnet.

Der 3,4-DABE enthaltende Strom wird vor der Hydroformylierung zum C₅-Acetat vorzugsweise einer Nachveresterung mit Acetanhydrid, Essigsäure oder Keten zugeführt, wobei der darin enthaltende 3,4-HABE-Anteil zu 3,4-DABE nachverestert wird.

Der gegebenenfalls einer Nachveresterung unterworfene, 3,4-DABE enthaltende Strom kann destillativ gereinigt oder unmittelbar der Hydroformylierung zum C₅-Acetat zugeführt werden. Zweckmäßigerweise entfernt man die aus dem Acetanhydrid freigesetzte Essigsäure bzw. das entstehende Reaktionswasser, falls die Veresterung mit Essigsäure durchgeführt wird.

Die Hydroformylierung erfolgt in bekannter Weise mit Kohlenmonoxid und Wasserstoff, bevorzugt in Gegenwart von Rhodiumcarbonyl-Komplexen als Katalysatoren, bei erhöhter Temperatur und erhöhtem Druck. Besonders bevorzugt wird die Hydroformylierung nach dem eingangs beschriebenen Verfahren der DE-A 19 45 479 durchgeführt.

Die Erfindung wird im folgenden anhand einer Figur und eines Ausführungsbeispiels näher erläutert.

### Es zeigen im einzelnen:

Figur 1 die Reaktionskinetik der Hydroformylierungsreaktion, d.h. den Umsatz U in Prozenten in Abhängigkeit von der Reaktionszeit t in Stunden für das erfindungsgemäße Verfahren (Kurve I) im Vergleich zum Stand der Technik (Kurve II) und

Figur 2 das Schema einer Destillationskolonne zur Auftrennung des aus der Acetoxylierung von 1,3-Butadien erhaltenen Stroms 1 zur Gewinnung eines 3,4-DABE enthaltenden Stroms 4 über einen Seitenabzug der Destillationskolonne.

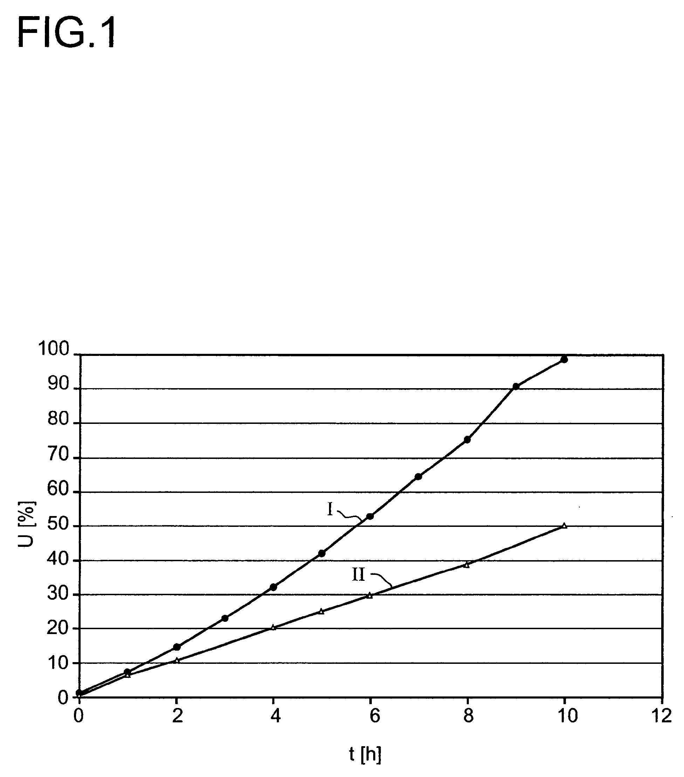

Im nachfolgenden Diagramm in Figur 1 ist die Kinetik der Hydroformylierungsreaktion von 3,4-DABE dargestellt, wobei die untere Kurve, II, für eine Hydroformylierungsreaktion aufgenommen wurde, bei der ein konventionell, aus den von Buten(2)diol(1,4) hergestelltes 3,4-DABE eingesetzt wurde, wogegen die obere Kurve, I, für eine Hydroformylierungsreaktion aufgenommen wurde, bei der, entsprechend dem erfindungsgemäßen Verfahren, von einem 3,4-DABE ausgegangen wurde, das durch destillative Abtrennung aus dem Axetoxylierungsprodukt von 1,3-Butadien und anschließender Nachveresterung des enthaltenen 3,4-HABE erhalten wurde.

Das Edukt für den Vergleichsversuch (Kurve II) wurde durch Acetylierung von Buten(2)diol(1,4) und anschließender Isomerisierung mit Eisessig und Kupfer-II-Acetat, entsprechend Beispiel 3 der DE-B 24 06 058 erhalten.

Beide Ausgangsmaerialien, das heißt sowohl für den Vergleichsversuch als auch für das Beispiel nach dem erfindungsgemäßen Verfahren enthielten ca. 95 bis 98 % 3,4-DABE sowie mehrere, nicht näher aufgeklärte Nebenkomponenten.

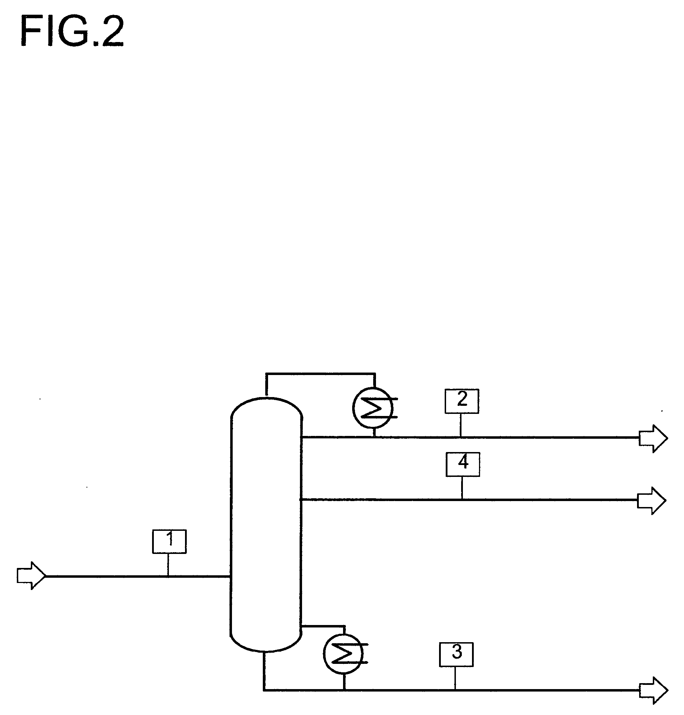

Demgegenüber wurde für die Hydroformylierungsreaktion nach dem erfindungsgemäßen Verfahren, deren Kinetik in Kurve I dargestellt ist, ein 3,4-DABE enthaltender Strom eingesetzt, der aus der katalytischen Acetoxylierung von 1,3-Butadien erhalten und anschließend destillativ aufgetrennt wurde. In der nachfolgenden Figur 2 ist die Auftrennung des Acetoxylierungsgemisches (Strom I) in eine Destillationskolonne, unter Gewinnung eines 3,4-DABE enthaltenden Stroms (4) dargestellt.

Die Destillationskolonne ist, wie üblich, mit einem Sumpfverdampfer und einem Kondensator am Kolonnenkopf ausgestattet, die schematisch dargestellt sind. Strom 2 bezeichnet das am Kolonnenkopf abgezogene Leichtsiedergemisch und Strom 3 das aus dem Kolonnensumpf abgezogene Schwersiedergemisch, das in weiteren Verfahrensschritten zum 1,4-Butandiol verarbeitet wird.

Die Zusammensetzung der Ströme in kg/kg ist in der nachfolgenden Tabelle aufgeführt.

| Strom Nr. | 1 | 2 | 3 | 4 |
|---|---|---|---|---|
| Essigsäure | 0,0290 | 0,9638 | 0,0000 | 0,0090 |
| 1,4-Diacetoxybuten-cis | 0,1140 | 0,0000 | 0,1318 | 31 ppm |
| 1,4-Diacetoxybuten-trans | 0,7290 | 0,0000 | 0,8427 | 5 ppm |
| 3,4-DABE | 0,0990 | 0,0020 | 0,0125 | 0,8325 |
| Monoacetoxybuten | 0,0010 | 0,0337 | 0,0000 | 179 ppm |
| 1,4-Hydroxyacetoxybuten | 0,0020 | 0,0000 | 0,0023 | 2 ppm |
| 3,4-HABE | 0,0180 | 0,0005 | 0,0014 | 0,1582 |
| Axetoxycrotonaldehyd | 0,0080 | 0,0000 | 0,0092 | 17 ppm |
| Menge bezogen auf Zulauf | 100% | 3% | 87% | 11% |

Der überwiegend 3,4-DABE enthaltende Strom 4 wurde mit Acetanhydrid nachverestert, destillativ gereinigt und anschließend mit einem Gehalt von ca. 95 bis 98 % DABE für die Hydroformylierungsreaktion zum C₅-Acetat eingesetzt.

Die Darstellung in Figur 1 zeigt, daß mit dem erfindungsgemäßen Verfahren ein Umsatz von ca. 50% nach etwa 5,5 Stunden erreicht wurde (Kurve I), wobei für denselben Umsatz von 50% nach dem Stand der Technik die beinahe doppelte Zeit, von 10 Stunden, erforderlich war (Figur II).

## Patentansprüche

1. Verfahren zur Herstellung von C₅-Acetat für die Vitamin A-Synthese durch Hydroformylierung von 3,4-Diacetoxy-Buten(1), (3,4-DABE) **dadurch gekennzeichnet, daß** man einen 3,4-DABE enthaltenden Strom einsetzt, den man durch Acetoxylierung von 1,3-Butadien mit Essigsäure und Luft in Gegenwart eines Katalysators und destillative Abtrennung des hierbei als Nebenprodukt gebildeten 3,4-DABE erhalten hat.

2. Verfahren nach Anspruch 1, **dadurch gekennzeichnet, daß** man den 3,4-DABE enthaltenden Strom als flüssigen Seitenstrom einer Destillationskolonne abtrennt.

3. Verfahren nach Anspruch 1 oder 2, **dadurch gekennzeichnet, daß** die Destillationskolonne bei einer Sumpftemperatur im Bereich von 110 bis 190°C, bevorzugt von 120 bis 150°C, besonders bevorzugt bei 130°C und einem Druck von 5 bis 200 mbar, insbesondere von 20 bis 50 mbar, besonders bevorzugt von 30 mbar, betrieben wird.

4. Verfahren nach einem der Ansprüche 1 bis 3, **dadurch gekennzeichnet, daß** destillativ ein 75 bis 90 Gew.-%, bevorzugt ca. 85 Gew.-% 3,4-DABE enthaltender Strom abgezogen wird.

5. Verfahren nach einem der Ansprüche 1 bis 4, **dadurch gekennzeichnet, daß** man 3,4-DABE mit Kohlenmonoxid und Wasserstoff in Gegenwart von Carbonylkomplexen des Rhodiums bei erhöhter Temperatur und unter erhöhtem Druck umsetzt.
